# EUROPÄISCHE PATENTSCHRIFT

(11) **EP 1 257 522 B1**
(45) Veröffentlichungstag und Bekanntmachung des Hinweises auf die Patenterteilung: **04.01.2006**
(21) Anmeldenummer: 01915183.6
(22) Anmeldetag: 01.02.2001
(51) Int. Cl.: C07C 209/78, C07C 211/50

(54) **VERFAHREN ZUR HERSTELLUNG VON DIAMINODIPHENYLMETHANEN**
METHOD FOR PRODUCTION OF DIAMINODIPHENYLMETHANES
PROCEDE DE PRODUCTION DE DIAMINODIPHENYLMETHANES

(30) Priorität: 14.02.2000 DE 10006452
(43) Veröffentlichungstag der Anmeldung: 20.11.2002
(73) Patentinhaber: Bayer MaterialScience AG, 51368 Leverkusen (DE)
(72) Erfinder: KLEIN, Stephan, 51467 Bergisch Gladbach (DE); GROTJOHANN, Dirk, 51377 Leverkusen (DE); MENDOZA-FROHN, Christine, 40699 Erkrath (DE); KOCH, Daniel, 47137 Duisburg (DE); MÜLLER, Heinz-Herbert, 47809 Krefeld (DE); PIRKL, Hans-Georg, 51377 Leverkusen (DE); UCHDORF, Rudolf, 47829 Krefeld (DE); WEGENER, Gerhard, 40822 Mettmann (DE)
(86) Internationale Anmeldenummer: PCT/EP2001/001083
(87) Internationale Veröffentlichungsnummer: WO 2001/058847

(56) Entgegenhaltungen:
- US-A- 4 039 581

## Beschreibung

Die vorliegende Erfindung betrifft ein Verfahren zur Herstellung von hoch monomerhaltigem Diaminodiphenylmethan (MDA) mit geringem ortho-Gehalt durch an festen Säuren katalysierte Umlagerung eines Kondensationsproduktes (sog. Aminal oder N,N-Diphenylmethylendiamin) aus Anilin und Formalin oder einem anderen geeigneten methylengruppenliefernden Agens, wie Trioxan oder p-Formaldehyd.

MDA (insbesondere das 4,4'-Isomere) ist ein hervorragend geeignetes Ausgangsmaterial, aus dem - ggf. nach weiterer Reinigung - durch Phosgenierung Diisocyanate erhalten werden, die ein wichtiges Rohprodukt beispielsweise für Polyurethansysteme darstellen. Daneben spielen auch die ausgehend vom MDA durch Hydrierung des aromatischen Ringes erhaltenen aliphatischen Systeme eine wichtige Rolle als Lackrohstoffe.

Unter den vielen denkbaren und in der Literatur beschriebenen Verfahren zur Herstellung von MDA ist die Erzeugung aus dem Anilin-Formaldehyd-Kondensationsprodukt (sog. Aminal) die wichtigste, weil wirtschaftlich vorteilhafteste. Je nach Variante wird entweder zunächst das Kondensationsprodukt hergestellt und dieses dann in Gegenwart von (zumeist) Mineralsäuren wie HCl umgelagert, oder aber die Kondensation bereits in Gegenwart von Säuren unter Umlagerungsbedingungen durchgeführt.

Die Nachteile solcher Verfahren im Hinblick auf die Synthese von Monomer-MDA lassen sich wie folgt zusammenfassen: Die Verfahren führen je nach Reaktionsbedingungen zu Gemischen aus überschüssigem Anilin, MDA-Monomer (2,2',- 2,4'- und 4,4'-Isomeres) sowie mehrkernigen Verbindungen (sog. Polymerbasen), aus denen das Monomer-MDA - ggf. nach Überführung in die entsprechenden Isocyanate - gewonnen werden kann. Eine Darstellung von fast polymerfreien MDA-Basen ist dabei nur in extremen Überschüssen von Anilin gegenüber dem methylengruppenliefernden Agens zu bewerkstelligen, was zu geringen Raum-Zeit-Ausbeuten und großen Anilinkreislaufströmen führt.

Ein weiterer Nachteil der Darstellung durch Katalyse mit Mineralsäuren ist der Anfall von salzhaltigen Abwässern, die bei der Neutralisation der Säure entstehen. Darüber hinaus führen wässrige Mineralsäuren zu Korrosionsproblemen bei den Anlagen.

Es gibt daher bereits eine ganze Reihe von Vorschlägen zur technischen Ausführung der Umlagerung auch an festen Säuren, um diese Nachteile zu umgehen.

Ein technisch realisierbares Verfahren zur MDA-Monomerbasenherstellung unter Vermeidung von Mineralsäuren muss jedoch z.B. folgende Voraussetzungen erfüllen:
a) Quantitative Umsätze: es muss ein intermediat- (Aminobenzylanilin-) freies Produkt erhalten werden, um die Phosgenierbarkeit zu gewährleisten (Diese können in der weiteren Verarbeitung des MDA zum MDI (Phosgenierung) extrem störend sein).
b) Isomerenverteilung: ähnlich wie bei den mineralsäurekatalysierten Verfahren muss in gewissem Umfang eine Steuerung der Produktzusammensetzung durch Variation der Prozessparameter möglich sein.
c) Standzeiten: ein technisch eingesetzter Katalysator muss bei hohen Raum-Zeit-Ausbeuten wirtschaftliche Standzeiten erreichen, bevor seine Aktivität durch eine Regenerierung wieder hergestellt werden kann.
d) Fremdsubstanzen: der eingesetzte Katalysator darf keine Spurenbestandteile in das Produkt abgeben, die Produktqualität negativ beeinflussen. Zudem sollte durch das Verfahren kein Fremdstoff, z.B. in Form eines systemfremden Lösungsmittels, in die Reaktionsmischung eingeschleppt werden.

Verschiedene Ansätze zur selektiven Gewinnung des reinen Zweikernmoleküls wurden bereits beschrieben, wobei zwischen (a) Methoden der stofflichen Trennung von Isomeren- und Homologengemischen und (b) der selektiven Synthese der Zweikernverbindungen unterschieden werden muss:
(a) Methoden der selektiven 4,4'-MDA-Kristallisation aus halogenierten Lösungsmitteln wurden zum Beispiel in US-A-4 172 847 beschrieben. Diese Aufarbeitung hat allerdings den Nachteil, dass in einem aufwendigen, zusätzlichen Verfahrensschritt ein systemfremdes Lösungsmittel verwendet werden muss, wodurch zusätzliche Stoffkreisläufe und Trennoperationen nötig werden.
(b) In US-A-4 011 278 wird von zahlreichen Umsetzungen polarer organischer Verbindungen an Zeolithe ZSM-5 und anderen Zeolithtypen berichtet. Dabei wird auch die Umsetzung von N-Alkylanilin mit Formaldehyd in Gegenwart von Zeolithkatalysatoren erwähnt, ohne jedoch Angaben zu Reaktionsbedingungen, Umsätzen und Selektivitäten zu machen.

Aus DE-A-2 202 500 ist bekannt, dass bei einer Aminalumlagerung an amorphen Silizium-Aluminium-Mischoxid-Crackkatalysatoren dann hohe Anteile von 4,4'-Isomeren gefunden werden, wenn die Reaktion bereits in der Gegenwart von ortho-Isomeren durchgeführt wird (die beispielsweise aus einem anderen Ansatz gewonnen wurden). In Gegenwart dieser Isomere werden keine zusätzlichen Anteile 2,2'- und 2,4'-MDA mehr erhalten, da diese nach ihrer primären Bildung bevorzugt zu höherfunktionellen, oligomeren MDA-Typen abreagieren. Somit wird dabei gewöhnlich ein hoher Polymerbasenanteil erhalten, der von dem gewünschten 4,4'-Isomeren abgetrennt werden muss. Zudem erfordert dieser Prozess als zusätzlichen Schritt die Rückführung gebildeter o-Isomere.

Vorteilhafter wäre es, schon während der Synthese des MDAs Bedingungen einzustellen, die möglichst hohe 4,4'-MDA-Anteile gewährleisten, wobei durch Synthese an festen Säuren gleichzeitig auch die oben angesprochene Salz- und Korrosionsproblematik umgangen werden kann.

So ist aus der oben genannten US-A-4 011 278 bereits bekannt geworden, dass beispielsweise an festen oxidischen Säuren, insbesondere aber an Zeolithen z.T. hohe Selektivitäten bzgl. der Zweikernverbindung und insbesondere zum 4,4'-MDA erhalten werden können.

EP-A-0 264 744 beschreibt die Kondensation von Anilin mit Trioxan oder freiem Formaldehyd und die Umlagerung in die MDA-Basen an festen, Bor-haltigen Zeolithen. Sowohl die simultane Kondensation und Umlagerung, als auch die Isolierung der Aminobenzylaniline und anschließende Umlagerung zu MDA wurden durchgeführt. Dabei werden zwar bei der Umlagerung der Aminobenzylaniline zu MDA hohe Monomer-Selektivitäten erhalten (ca. 90 m-% Zweikernisomere im anilinfreien Gemisch), jedoch wird zum einen kein vollständiger Umsatz erreicht, zum anderen wird bevorzugt in Benzol als Lösungsmittel gearbeitet.

Es ist auch bereits versucht worden, die nach dem Stand der Technik in mehreren Schritten, beispielsweise in zwei Schritten, ablaufende Umlagerung des Aminals über das Aminobenzylanilin zum MDA an festen Säuren in mehr als einem Schritt ablaufen zu lassen. US-A-4 039 581 beschreibt die Umlagerung eines Aminals aus Formaldehyd und Anilin an festen Säuren, wobei es zunächst einer Trocknung unterworfen wird und anschließend an z.B. Zeolithen in mehreren Reaktionsstufen - gekennzeichnet durch Temperaturstufen - umgelagert wird. Dabei wird eine Temperatur von 100°C allerdings nicht überschritten, weil von der Annahme ausgegangen wird, hohe Temperaturen in Gegenwart von Wasser seien selektivitätsschädigend. Unter diesen Bedingungen ist eine vollständige Umlagerung der Aminobenzylanilin-Intermediate zu den MDA-Basen nicht zu erreichen. Als Produkt wird ein MDA mit einem Zweikemgehalt von ca. 90 m-% im anilinfreien Gemisch erhalten.

Die EP-A-0 329 367 beschreibt die Umlagerung eines getrockneten Aminals an zeolithischen Katalysatoren zum Zwecke der selektiven Herstellung von Zweikern-MDA. An dealuminierten HY-Zeolithen und deren fluorierten Abkömmlingen wird ein Aminal bei 120°C isotherm zu einem MDA-Gemisch umgelagert, das zwar zu ca. 94 m-% (bzgl. anilinfreier Lösung) aus Zweikern-MDA besteht, aber durch unvollständigen Umsatz der Intermediate zu den MDA-Basen gekennzeichnet ist. Zudem entstehen ca. 5 m-% Polymerbasen.

Der vorliegenden Erfindung lag daher die Aufgabe zugrunde, ein Verfahren zu finden, nach dem es entgegen der in der Literatur vertretenen Meinung möglich ist, die Umwandlung von Aminal in polymeiarmes Zweikern-MDA unter Einsatz von festen sauren Katalysatoren durchzuführen und dabei die Vorteile der Katalyse mit Feststoffen bei für technische Verfahren geforderten, ausreichend langen KatalysatorStandzeiten zu nutzen, ohne dabei nennenswerte Restmengen an polymeren MDA-Basen oder nicht vollständig umgelagerter Intermediate (Aminobenzylaniline) im Endprodukt zu erhalten.

Erfindungsgemäß wird diese Aufgabe durch ein Verfahren zur Herstellung von Diaminodiphenylmethanen gelöst, in welchem durch Umlagerung eines Kondensationsproduktes aus Anilin und Methylengruppen lieferndem Agens, z.B. Formaldehyd, in welchem ein getrocknetes Kondensat von Anilin und dem Methylengruppen lieferndem Agens eines molaren Verhältnisses von Anilin zu Methylengruppen lieferndem Agens von 1,7 bis 100 in Gegenwart festerfester, saurer Katalysatoren unter produktschonenden Bedingungen zu polymerarmem Diaminodiphenylmethan mit überwiegendem 4,4'-Isomerenanteil umgesetzt wird, das dadurch gekennzeichnet ist, dass Anilin eingesetzt wird, das weitgehend frei von aliphatischen Aminen ist.

Polymerarmes Diaminodiphenylmethan im Sinne der Erfindung besteht zu mindestens 80 Gew.-%, vorzugsweise 85 Gew.-%, besonders bevorzugt mindestens 90 Gew.-%, aus Zweikemverbindungen, wovon der ortho-Isomeren-Anteil maximal 20 Gew.-%, vorzugsweise maximal 18 Gew.-%, besonders bevorzugt maximal 16 Gew.-% und der Anteil von 4,4'-Isomeren minimal 80 Gew.-%, vorzugsweise minimal 82 Gew.-%, besonders bevorzugt minimal 84 Gew.-% ausmacht.

Hieraus ergibt sich, dass der Anteil an 4,4'-Isomeren am anilinfreien Gesamtgemisch zwischen 64 und 100 Gew.-% liegt. Hiermit ist der überwiegende 4,4'-Isomerenanteil im polymerarmen Diaminodiphenylmethan definiert.

Dieses Verfahren lässt sich in idealisierter Weise am folgenden Schema illustrieren: 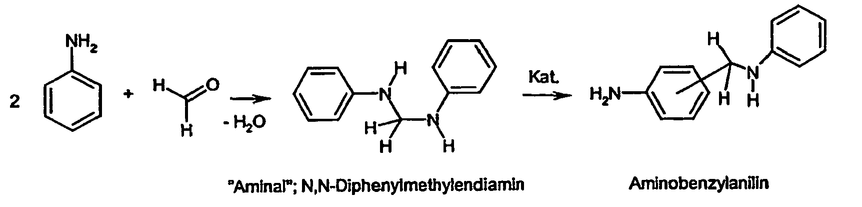 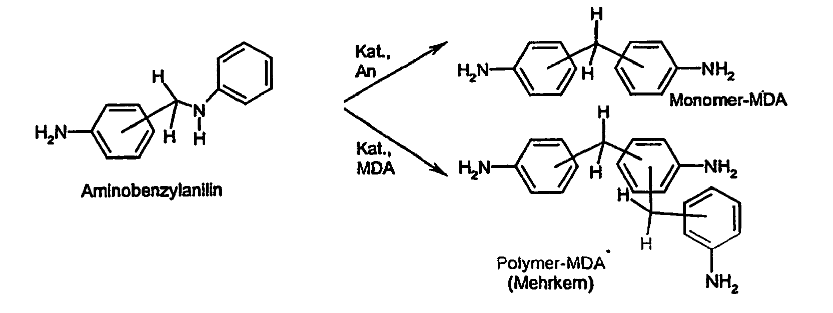 Methylengruppenliefemde Verbindungen im Sinne der Erfindung sind neben wässriger Formaldehyd-Lösung beispielsweise auch p-Formaldehyd und Trioxan. Diese werden mit Anilin zur Reaktion gebracht, wobei ein Kondensationsprodukt entsteht, das mit dem Aliasnamen Aminal bezeichnet werden kann und hauptsächlich aus N,N-Diphenylmethylendiamin besteht. Dieses Kondensationsprodukt wird zunächst entwässert, bevor die Weiterreaktion katalysiert durchgeführt wird.

Prinzipiell kann die Reaktion zum Aminal auch bereits in Gegenwart eines die Umlagerung zu ABA und/oder den MDA-Isomeren bedingenden Katalysators durchgeführt werden. Das bei der Kondensationsreaktion freiwerdende Wasser hat jedoch einen aktivitäts- und selektivitätssenkenden Einfluss auf den Katalysator, weshalb die sukzessive Variante (Aminalreaktion → Entwässerung → Umlagerung) bevorzugt wird.

Die Aminalreaktion wird bevorzugt in kontinuierlicher Weise durchgeführt, in dem Anilin und Formaldehydlösung im molaren Verhältnis Anilin zu Formaldehyd von 1,7 - 100, bevorzugt von 2 bis 50, besonders bevorzugt von 4 bis 20, in einen Reaktor dosiert werden, aus dem eine dem Zustrom volumengleiche Reaktionsmenge kontinuierlich entnommen und der Phasentrennung zugeführt wird. Denkbar ist jedoch auch die dis- oder semikontinuierliche Durchführung, bei der Anilin und Formalin im gewünschten Mischungsverhältnis in einen gerührten Satzreaktor (Batchreaktor) dosiert werden, aus dem das ausreagierte Aminal dann der Trocknung zugeführt wird.

Die Entwässerung kann sowohl über in der Technik übliche Trockenmittel (z.B. Molekularsieb) kontinuierlich oder absatzweise erfolgen oder beispielsweise azeotrop durch kontinuierliche oder absatzweise Destillation (Entwässerung unter Zuhilfenahme des bereits im System vorhanden Anilins). Das dabei gegebenenfalls mit abgeführte Anilin kann zweckmäßigerweise bereits zu Beginn der Aminalreaktion im Überschuss zugesetzt werden, um nach der destillativen Trocknung das gewünschte Verhältnis von Anilin zu Aminal vorliegen zu haben.

Das für die Umlagerung erwünschte Verhältnis Anilin / Formaldehyd (A/F) kann bereits in der Aminalreaktion, gegebenenfalls unter Berücksichtigung der Trocknungsverluste, eingestellt werden. Es besteht jedoch prinzipiell auch die Möglichkeit, die Aminalreaktion und Aminaltrocknung bei niedrigem molaren A/F von 1 bis 5 durchzuführen und erst unmittelbar vor der Umlagerung mit reinem, trockenen Anilin auf den erwünschten Wert von 4 bis 20 einzustellen. Letzteres führt auf den Stufen Aminalreaktion und -trocknung zu kleineren Apparaten und damit geringeren Investitionskosten. Für diese Aufstockung nach Kondensation kann auch aus der Aufarbeitung des Reaktionsgemisches zurückgewonnenes Anilin (Rückanilin) verwendet werden, welches im Falle von Anilinüberschussfahrweisen aus dem komplett umgelagerten MDA zurückgewonnen wird.

Die destillative Trocknung wird bevorzugt kontinuierlich und unter vermindertem Druck durchgeführt, um so das Kondensationsprodukt der geringstmöglichen thermischen Beanspruchung auszusetzen.

Vorzugsweise wird die Entwässerung bis zu einem Gehalt von weniger als 1 000 ppm Wasser, besonders bevorzugt von weniger als 500 ppm Wasser, durchgeführt.

Die katalysierte Umlagerung des Kondensationsproduktes (Aminal) muss vollständig zum gewünschten MDA erfolgen. Vollständiger Umsatz gilt als erreicht, wenn die Zwischenprodukte bis auf eine Restkonzentration von 0 bis <500 mg/kg, bevorzugt <200 mg/kg, an ABA abreagiert sind.

Da die Folgeprodukte (d.h. unterschiedliche Isomerenzusammensetzungen) des MDAs in verschiedenen Anwendungen eingesetzt werden, ist eine Flexibilität in der Isomerenzusammensetzung für die industrielle Anwendung von besonderer Bedeutung. Die Steuerung der Isomerenzusammensetzung lässt sich wesentlich durch Variation der Reaktionstemperatur und des Katalysatortyps erreichen.

Man unterscheidet deshalb die erste, selektivitätsgebende Reaktionsphase (a) und die der Umsatzvervollständigung dienende Phase (b), wobei bei ausgewähltem Katalysatortyp das Verhältnis der Temperatur in den Reaktionsphasen über die Endzusammensetzung bzgl. der o- und p-Isomerenanteile entscheidet. Je niedriger die Temperatur in Phase (a) zwischen und 70°C gewählt wird, umso höher ist der erreichbare p-Isomerenanteil. Umgekehrt führen hohe Temperaturen in Phase (a) von 70 bis 200°C zu hohen o- Isomerenanteilen im Reaktionsgemisch.

In der zweiten Reaktionsphase (b) wird die Temperatur auf hohem Niveau geführt, bevorzugt bei 100 bis 200°C, um die Reaktion vollständig abzuschließen und die Zwischenprodukte abzubauen.

Die erfindungsgemäß eingesetzten Katalysatoren müssen diesen Anforderungen gerecht werden:

Es werden anorganische, bevorzugt oxidische, besonders bevorzugt silikatische Katalysatoren eingesetzt.

So wird beispielsweise als Katalysator ein kommerziell erhältlicher Zeolith vom Typ Y (Faujasit) mit einem Modul (SiO₂ / Al₂O₃) von 5 bis 200 eingesetzt, dem zum Zwecke der Formgebung ein Binder (z.B. Aluminiumoxid) zugegeben werden kann. Die Katalysatoren liegen bevorzugt in der H⁺-Form vor. Dies kann gegebenenfalls durch die bekannten Methoden (Säurebehandlung, Ammoniumionenaustausch mit anschließender thermischer Behandlung) erreicht werden.

Die Katalysatoren können prinzipiell sowohl in Pulverform, als auch in stückiger Form eingesetzt werden, wobei zur Formgebung z.B. die technisch üblichen Verfahren der Tablettierung, Granulierung oder Extrusion auch unter Zuhilfenahme von Formgebungshilfsmitteln eingesetzt werden können. Für die technische Anwendung im kontinuierlichen Verfahren wird der Katalysator zum Betrieb fester Katalysatorbetten bevorzugt nach Formgebung appliziert. Die Katalysatoren werden bei absatzweisem Betrieb bevorzugt in Mengen von 0,1 - 1000 Gew.-% bzgl. katalysatorfreiem Reaktionsgemisch, bei kontinuierlichem Betrieb bevorzugt in Mengen von 0,01 bis 100 kg Kat / (kg Aminal · h), besonders bevorzugt 0,1 bis 10 kg Kat/ (kg Animal · h), eingesetzt.

Im Verfahrensverlauf können auch hinsichtlich Typ, Geometrie etc. verschiedene Katalysatoren eingesetzt werden.

Bevorzugt wird das erfindungsgemäße Verfahren in Abwesenheit von Lösungsmitteln durchgeführt.

Es wurde auch gefunden, dass die geeigneten anorganischen, wie beispielsweise zeolithischen Katalysatoren durch im Anilin enthaltene Anteile von aliphatischen Aminen stark desaktiviert werden können. So enthält technisches Anilin erhebliche Anteile dieser Nebenprodukte (z. B. Cyclohexylamin, Dicyclohexylamin), die bereits zu einer signifikanten Desaktivierung führen. Technisches Anilin wird industriell durch Destillation von Roh-Anilin gewonnen.

Für das erfindungsgemäße Verfahren werden deshalb Anilinqualitäten eingesetzt, die weitgehend frei von aliphatischen Aminen als Neben- und Spurenbestandteilen (z.B. Cyclohexylamin, Dicyclohearylamin) sind. Für das erfindungsgemäße Verfahren wird bevorzugt Anilin mit einer Reinheit ≥ 99,5 % verwendet, wobei Anilin-Qualitäten mit aliphatischen Amin-Gehalten (z. B. Cyclohexylamin, Dicyclohexylamin) von weniger als 100 ppm bevorzugt sind. Besonders bevorzugt werden Anilin-Qualitäten mit aliphatischen Amin-Gehalten von 0 bis < 25 ppm eingesetzt. Die Entfernung der aliphatischen Amine aus Anilin kann vorteilhaft durch Adsorption, vorzugsweise Chemisorption, oder saure Wäsche erfolgen.

Die erfindungsgemäße Umlagerung wird mit dem getrockneten Aminal beispielsweise so durchgeführt, dass das aus der Trocknung erhaltene Produkt mit dem festen Katalysator in Suspension in Kontakt gebracht wird. Dabei kann die Umlagerung absatzweise oder kontinuierlich im Rührkessel, in einer Rührkesselkaskade, im Rohrreaktor (z.B. Fest- oder Fließbettreaktor) oder einer Kombination davon durchgeführt werden. Vorteilhaft wird mit seriellen Katalysatorfestbetten gearbeitet. Dabei wird in Abhängigkeit vom eingesetzten Katalysator im Temperaturbereich von 20 - 70°C, bevorzugt 40 - 60°C, zunächst ein Gemisch von Aminobenzylanilinen, Anilin und zu kleinen Teilen Diaminophenylmethanen erhalten. Das Reaktionsgemisch wird dazu vorzugsweise über das Katalysatorfestbett gepumpt, wobei typischerweise Verweilzeiten von 0,2 bis 2 Stunden eingestellt werden. Die für einen ausgewählten Katalysator und ein gewünschtes Isomerenverhältnis bei den erhaltenen Aminobenzylanilinen optimale Temperatur wird durch Vorversuche auf einfache Weise ermittelt.

Im weiteren Verlauf wird bei gesteigerter Temperatur von 70 -140°C, bevorzugt 90 - 130°C, die weitere Umlagerung der Aminobenzylaniline zu den MDA-Isomeren durchgeführt, wobei typischerweise Verweilzeiten von 0,2 bis 2 Stunden eingestellt werden. Verwendet wird dazu ebenfalls ein Katalysatorbett als besonders bevorzugte Ausführungsform, auch alle anderen oben erwähnten Techniken sind ebenfalls einsetzbar.

Erforderlichenfalls kann im weiteren Verlauf bei weiter gesteigerter Temperatur von 130 - 200°C, bevorzugt 140 - 175°C, die Umlagerung eventuell restlicher Aminobenzylaniline zu MDA-Isomeren durchgeführt werden, ohne dass dabei MDA-Polymere in nennenswerter Menge gebildet werden oder erhebliche Mengen an 4,4'-MDA zu ortho-Isomeren isomerisiert werden. Dazu werden typischerweise Verweilzeiten von 0,02 bis 2 Stunden, vorzugsweise 0,1 bis 1 Stunde, an einem weiteren Katalysatorbett eingestellt.

Nach Beendigung der Reaktion kann das nach dem erfindungsgemäßen Verfahren erhaltene Reaktionsgemisch in einem Schritt (c) so aufgearbeitet werden, dass in kontinuierlicher oder absatzweiser Form das im Gemisch gegebenenfalls enthaltene, überschüssige Anilin durch bekannte Verfahren wie beispielsweise Destillation oder Kristallisation von den MDA-Isomeren getrennt und recycliert werden kann. Die MDA-Isomere werden dann der nachfolgenden Phosgenierung zugeführt.

Es ist überraschend, dass nach diesem Verfahren polymerarme MDA-Typen mit hohem Anteil an 4,4'-Isomerem zugänglich sind und auch dass dafür handelsübliche Y-Zeolithe als Katalysatoren eingesetzt werden können, ohne diese zuvor einer Modifizierung, z.B. einer Fluorierung unterziehen zu müssen. Insbesondere ist überraschend, dass durch Variation der Temperatur und/oder des Katalysatortyps in der ersten Reaktionsphase (a) der p-Isomerengehalt gezielt gesteuert werden kann und der o-Isomerengehalt (2,4'-MDA und 2,2'-MDA) sich auf maximal 20 Gew.-%, vorzugsweise maximal 18 Gew.-%, besonders bevorzugt maximal 16 Gew.-% (bezogen auf die Gesamtmenge an Zweikernverbindungen) minimieren lässt.

Die aus dem erfindungsgemäßen Verfahren erhaltenen Produkteigenschaften werden durch die Kombination der Verfahrensparameter Temperatur, A/F-Verhältnis, insbesondere aber durch die Wahl des Zeolithen mit im gewählten Temperaturfenster optimaler Aktivität eingestellt, wie in den folgenden Beispielen gezeigt wird.

Die vorliegende Erfindung wird durch die folgenden Beispiele illustriert, ist aber in keiner Weise als auf diese Beispiele beschränkt zu verstehen. Die Beispiele sollen den Fachmann insbesondere dabei unterstützen, geeignete Katalysatoren für die jeweils gewünschte Ausgestaltung des Verfahrens auszuwählen sowie die für einen ausgewählten jeweiligen Katalysator die optimale Temperaturführung zu ermitteln.

### Beispiele

### Beispiel 1

300 g Anilin und 33,6 g wässrige Formaldehydlösung (32 Gew.% Formaldehyd in Wasser) entsprechend einem Molverhältnis von A/F = 9 werden in einem Satzreaktor unter Schutzgas zusammengegeben, wobei bei einer Temperatur von 60°C spontan und unkatalysiert die Bildung des Aminals einsetzt. Nach Überführung des Reaktionsgemisches in einen Scheidetrichter setzt eine Phasentrennung ein, und die organische Phase wird abgetrennt und einer weiteren Trocknung unterworfen.

Diese Trocknung wurde auf verschiedene Weise durchgeführt wobei die gewählte Art keinen nennenswerten Einfluss auf das Endergebnis hatte:
a) mittels Trockenmittel:
   die feuchte Aminalphase (170 g) wird bei ca. 60 - 80°C mit 50 g eines Trockenzeolithen (Molsieb 4 A, Bayer AG) versetzt und die über dem Molsieb stehende Lösung unter Schutzgas für ca. 1 h gerührt. Die so getrocknete organische Phase ist klar und von hellbrauner Farbe und hat einen durchschnittlichen Wassergehalt nach Karl-Fischer-Methode von < 0,05%.
b) mittels Batchdestillation:
   Die wässrige Aminalemulsion (527,5 g) mit einem Wassergehalt von ca. 5 Gew.-% wird zur Entwässerung bei einem Druck von 100 hPa über einen Wasserabscheider unter Rückfluss auf einem 1000 ml-Kolben diskontinuierlich destilliert. Zunächst wird bei einer Kopftemperatur von ca. 50°C destilliert, welche im Verlauf abnehmender Wasserkonzentration im Aminal auf eine maximale Kopftemperatur von 110 bis 115°C bei einer Sumpftemperatur von 117 bis 120°C gesteigert werden muss. Im Wasserabscheider des Kondensates werden Wasser- und Amilinphase getrennt und die Amilinphase in den Kolben zurückgespeist. In der klaren und blassbraunen Lösung des Sumpfes (460 g) wurde nach der Karl-Fischer-Methode ein durchschnittlicher Wassergehalt von ca. 0,04% ermittelt.
c) mittels kontinuierlicher Destillation:
   Die wässrige Aminalemulsion mit einem Wassergehalt von ca. 5 Gew.% wird zur Entwässerung in einer Rektifizierkolonne kontinuierlich destilliert. Dabei wird das feuchte Aminal der Kolonne bei einem Kopfdruck von 100 mbar z.B. und einer einer entsprechenden Kopftemperatur von 48°C als Feed mit 100 g/h zugeführt, so dass kontinuierlich über Kopf ein Wasser-Anilin-Azeotrop mit 18 g/h und am Kolonnenfüß das nahezu wasserfreie Aminal mit 82 g/h abgenommen werden können. In dem klaren und blassbraunen Sumpfablauf wurde nach Karl-Fischer-Methode ein durchschnittlicher Wassergehalt von ca. 0,04% ermittelt.

### Beispiel 2

a) Das gemäß Beispiel 1 a) hergestellte und getrocknete Aminal wird in einer diskontinuierlichen Versuchsanordnung an einem bei 300°C über 15 h aktivierten, kommerziellen H-Y-Zeolith-Extrudat (DEGUSSA WESSALITH® DAY F 20, Degussa AG) umgesetzt. Auf 100 g trockenes Aminal werden dabei 15 g des aktivierten Katalysatorformkörpers eingesetzt. In einer temperaturgestuften Fahrweise wird nun zunächst bei 50°C für 6 h und anschließend bei 130°C für 4 h die Reaktion durchgeführt und das Reaktionsgemisch mittels HPLC analysiert. In der Hochtemperaturphase werden innerhalb der analytischen Nachweisgrenze die letzten Intermediatreste (Aminobenzylaniline) abgereichert. Es wird bezogen auf das anilinfreie Gemisch ein MDA der Zusammensetzung 98 m-% Monomer-MDA und 2 m-% mehrkernige Verbindungen erhalten. Der o-Isomerengehalt beträgt 14 m-%.

b) Analog zum Beispiel 2 a) werden dieselben Einsatzmengen unter vergleichbaren apparativen Bedingungen zur Reaktion gebracht, wobei jedoch die Temperatur nicht gestuft wird, sondern die Reaktion isotherm bei 130°C geführt wird. Nachdem die letzten Intermediatreste (Aminobenzylaniline) innerhalb der analytischen Nachweisgrenze vollständig abgereichert sind, wird bezogen auf das anilinfreie Gemisch ein MDA der Zusammensetzung 91 m-% Monomer-MDA und 9 m-% mehrkernige Verbindungen erhalten. Der o-Isomerengehalt beträgt 21 m-%.

### Beispiel 3

Das gemäß Beispiel 1 a) hergestellte und getrocknete Aminal wird in einem Umpump-Versuch an einem kommerziellen, zuvor bei 300°C über 15 h aktivierten H-Y-Zeolith-Extrudat (WESSALITH® DAY F 20, Degussa AG) umgesetzt. Das trockene Aminal wird dabei über eine Schüttung des aktivierten Katalysators geleitet, welche sich in einem mantelbeheizten Glasrohr befindet. In einem ersten Reaktor wird nun zunächst bei 50°C und einem Volumenstrom von ca. 60 ml/min das Aminal über das Festbett aus 50 g Katalysator geleitet und von einer Pumpe wieder in den Reaktor zurückgeführt, wobei eine Gesamtlaufzeit von 90 Min. erreicht wird. Dann wird das Reaktionsgemisch in einen analog aufgebauten Reaktor bei 130°C für 45 Min. über das Katalysatorbett gepumpt. Schließlich erfolgt die letzte Temperung bei 150°C für weitere 30 Min. in einem analogen Aufbau, wobei diese letzte Phase lediglich der Sicherheit dient, die unerwünschten Aminobenzylanilin-Intermediate vollständig abzureichern. In der Hochtemperaturphase werden ebenfalls innerhalb der analytischen Nachweisgrenze die letzten Intermediatreste (Aminobenzylaniline) abgereichert. Es wird ein MDA der Zusammensetzung 97 m-% Monomer-MDA und 3% mehrkernige Verbindungen erhalten. Der o-Isomerengehalt beträgt 14%.

### Beispiel 4

Analog Beispiel 3 wird jede Stufenfolge (50 → 130 → 150°C) sukzessive über 20 Reaktionszyklen in identischer Art und Weise mit zuvor frisch zubereitetem Aminal betrieben, wobei folgende Umsätze (in % der theoretischen Ausbeute) und - selektivitäten als Funktion der Laufzeit beobachtet wird.

| | **Lauf** | | | |
|---|---|---|---|---|
| | **3** | **7** | **13** | **20** |
| 4,4'-MDA: | 84,1 % | 83,4 %. | 84,0 % | 84,8 % |
| 2,4-MDA: | 13,5 % | 12,7 % | 12,9 % | 14,6 % |

### Beispiel 5

Das gemäß Beispiel 1 b) hergestellte und getrocknete Aminal wird an einem zuvor bei 300°C über 15 h aktivierten, kommerziellen H-Y-Zeolith-Extrudat (WESSALITH® DAY F 20, Si/Al = 23, Degussa AG) umgesetzt. Hierzu wird die trockene Aminallösung bei einem Massefluss von 180 g/h über eine Schüttung des Katalysators (72 g) geleitet, welcher sich in einem mantelbeheizten Glasrohr (200x30 mm) befindet. Bei dieser Auslegung des Katalysatorbettes ist unter der gewählten Reaktionsemperatur von *T* = 50°C lediglich eine teilweise Umlagerung der eingesetzten Aminallösungen zu erwarten (ca. 50% d. Th.). Zur Quantifizierung der Katalysatoraktivität in Abhängigkeit von der Reaktionszeit werden dann dem auslaufenden Stoffstrom in regelmäßigen Zeitabständen Proben entnommen. Diese werden durch HPLC hinsichtlich des Umsatzes analysiert. In Abhängigkeit vom Gehalt aliphatischer Amine im eingesetzten Anilin werden folgende Aminalumsätze zu den entsprechenden Umlagerungsprodukten (Aminobenzylaniline, MDA) nach einer Katalysatorstandzeit von 24 h ermittelt:

| **aliphatische Amine (ppm)** | **Umsatz (% d.Th.)** |
|---|---|
| <5 | 62% |
| 15 | 43 % |
| 60 | 23 % |
| 1000 | < 1 % |

### Beispiel 6

Analog zu dem im Beispiel 5 beschriebenen Versuch werden mehrere Festbetten mit Katalysatorschüttung seriell mit ansteigender Temperaturen betrieben. Hierzu wird die trockene Aminallösung bei einem Massefluss von 180 g/h über drei in Reihe geschaltete Schüttungen von je 72 g des Katalysators WESSALITH® DAY F 20, Degussa AG geleitet, welche sich in jeweils in mantelbeheizten Glasrohren (200x30 mm) bei einer Temperatur von 50°C befinden. Danach strömt das Reaktionsgemisch durch zwei in Reihe befindliche Festbetten analoger Bauart (2x72 g desselben Katalysators), die bei 130°C betrieben werden. Schließlich wird ein auf 150°C temperiertes Festbett analogen Aufbaus (72 g desselben Katalysators) durchströmt. Zur Quantifizierung der Katalysatoraktivität in Abhängigkeit von der Reaktionszeit werden dem auslaufenden Stoffstrom in regelmäßigen Zeitabständen Proben entnommen. Diese werden durch HPLC hinsichtlich des Umsatzes analysiert.

Bei Verwendung von Anilin mit einem Cyclohexylamingehalt von ca. 15 ppm wird so über eine kontinuierliche Gesamtbetriebsdauer von 5 Tagen bezogen auf das anilinfreie Gemisch unverändert ein MDA der Zusammensetzung 98 m-% Monomer-MDA und 2 m-% mehrkernige Verbindungen erhalten. Der o-Isomerengehalt beträgt 10 m-%.

## Patentansprüche

1. Verfahren zur Herstellung von Diaminodiphenylmethanen durch Umlagerung eines Kondensationsproduktes aus Anilin und Methylengruppen lieferndem Agens, in welchem ein getrocknetes Kondensat von Anilin und dem Methylengruppen lieferndem Agens eines molaren Verhältnisses von Anilin zu Methylengruppen lieferndem Agens von 1,7 bis 100 in Gegenwart fester, anorganischer, saurer Katalysatoren zu Diaminodiphenylmethan mit einem Anteil an 4,4'-Isomeren am Anilin-freien Gesamtgemisch von zwischen 64 und 100 Gew.-% umgesetzt wird, wobei im Temperaturbereich von 20-70°C zunächst ein Gemisch von Aminobenzylanilinen, Anilin und Diaminophenylmethanen erhalten wird und im weiteren Verlauf bei Temperaturen von 70-200°C die weitere Umlagerung der Aminobenzylaniline durchgeführt wird, **dadurch gekennzeichnet, dass** Anilin eingesetzt wird, das weniger als 100 ppm aliphatische Amine enthält.

2. Verfahren nach Anspruch 1, **dadurch gekennzeichnet, dass** die Umlagerung in mindestens zwei Stufen bei zwischen den Stufen steigender Temperatur durchgeführt wird.

3. Verfahren nach Ansprüchen 1 bis 2, **dadurch gekennzeichnet, dass** Anilin eingesetzt wird, das weniger als 25 ppm aliphatische Amine enthält.

4. Verfahren nach Ansprüchen 1 bis 3, **dadurch gekennzeichnet, dass** als Katalysatoren pulverförmige Zeolithe oder Zeolith-Formkörper in der H⁺-Form eingesetzt werden, die als Suspension oder als Festbetten eingesetzt werden.

5. Verfahren nach Ansprüchen 1 bis 4, **dadurch gekennzeichnet, dass** die Umlagerung absatzweise im Rührkessel, kontiniuerlich im Rührkessel, in einer Rührkesselkaskade, im Rohrreaktor, im Festbettreaktor, im Fließbettreaktor oder einer Kombination davon durchgeführt wird.

6. Verfahren nach Anspruch 1 bis 5, **dadurch gekennzeichnet, dass** zur Umlagerung ein Aminal oder eine Lösung hiervon eingesetzt wird, welches weniger als 1000 ppm Wasser enthält.

7. Verfahren nach Ansprüchen 1 bis 6, **dadurch gekennzeichnet, dass** FAU-Zeolithe eingesetzt werden.

## Claims

1. Process for the production of methylenedianilines by the rearrangement of a condensation product consisting of aniline and a methylene group-supplying agent, wherein a dried condensate of aniline and the methylene group-supplying agent having a molar ratio of aniline to methylene group-supplying agent of 1.7 to 100 is reacted in the presence of solid, inorganic, acid catalysts to methylenedianiline with a content of 4,4'-isomers in the aniline-free overall mixture of between 64 and 100 wt.%, wherein a mixture of aminobenzylanilines, aniline and diaminophenylmethanes is first obtained in a temperature range of 20 to 70°C, and in the continuing process the further rearrangement of the aminobenzylanilines is performed at temperatures of 70 to 200°C, **characterised in that** aniline is used that contains less than 100 ppm aliphatic amines.

2. Process according to claim 1, **characterised in that** the rearrangement is performed in at least two stages at a temperature that increases between the stages.

3. Process according to claims 1 to 2, **characterised in that** aniline is used that contains less than 25 ppm aliphatic amines.

4. Process according to claims 1 to 3, **characterised in that** powdered zeolites or zeolite mouldings in the H⁺ form, which are used in suspension or as fixed beds, are used as catalysts.

5. Process according to claims 1 to 4, **characterised in that** the rearrangement is performed batchwise in a stirred-tank reactor, continuously in a stirred-tank reactor, in a series of stirred-tank reactors, in a tubular reactor, in a fixed-bed reactor, in a fluidised-bed reactor or in a combination thereof.

6. Process according to claim 1 to 5, **characterised in that** an aminal or a solution thereof that contains less than 1000 ppm water is used for the rearrangement.

7. Process according to claims 1 to 6, **characterised in that that** FAU zeolites are used.

## Revendications

1. Procédé pour la préparation de diaminodiphénylméthanes par transposition d'un produit de condensation de l'aniline et d'un réactif fournissant des groupes méthylène, dans lequel on convertit un condensat sec d'aniline et du réactif fournissant les groupes méthylène à un rapport molaire aniline/réactif fournissant les groupes méthylène de 1,7 à 100 en présence de catalyseurs minéraux solides acides, en diaminodiphénylméthane contenant l'isomère 4,4' en proportion de 64 à 100 % du poids du mélange total exempt d'aniline, la réaction donnant d'abord dans l'intervalle de température de 20 à 70°C un mélange d'aminobenzylanilines, d'aniline et de diaminodiphénylméthanes sur lequel on poursuit, à des températures de 70 à 200°C, la transposition des aminobenzylanilines, ce procédé **se caractérisant en ce que** l'on met en oeuvre une aniline contenant moins de 100 ppm d'amines aliphatiques.

2. Procédé selon la revendication 1, **caractérisé en ce que** la transposition est réalisée en au moins deux stades opératoires à des températures croissant entre les divers stades opératoires.

3. Procédé selon les revendications 1 à 2 **caractérisé en ce que** l'on met en oeuvre une aniline contenant moins de 25 ppm d'amines aliphatiques.

4. Procédé selon les revendications 1 à 3, **caractérisé en ce que** l'on utilise en tant que catalyseurs des zéolithes en poudre ou à l'état d'objets façonnés sous la forme H⁺, qu'on met en oeuvre à l'état de suspension ou de lits fixes.

5. Procédé selon les revendications 1 à 4, **caractérisé en ce que** la transposition est réalisée en discontinu dans un récipient équipé d'un dispositif d'agitation, en continu dans un récipient équipé d'un dispositif d'agitation, dans une série de récipients équipés de dispositifs d'agitation, dans un réacteur tubulaire, dans un réacteur à lit fixe, dans un réacteur à lit fluidisé ou dans une combinaison de tels appareils.

6. Procédé selon les revendications 1 à 5, **caractérisé en ce que** pour la transposition, on met en oeuvre un aminal ou une solution d'aminal contenant moins de 1000 ppm d'eau.

7. Procédé selon les revendications 1 à 6, **caractérisé en ce que** l'on utilise des zéolithes du type faujasite.
